# FASCICULE DE BREVET EUROPEEN

(11) **EP 3 017 856 B1**
(45) Date de publication et mention de la délivrance du brevet: **09.01.2019**
(21) Numéro de dépôt: 15192141.8
(22) Date de dépôt: 29.10.2015
(51) Int. Cl.: B01D 53/047, C01B 13/02

(54) **DISPOSITIF DE PRODUCTION D'OXYGÈNE PURIFIÉ**
VORRICHTUNG ZUR HERSTELLUNG VON GEREINIGTEM SAUERSTOFF
DEVICE FOR PRODUCING PURIFIED OXYGEN

(30) Priorité: 07.11.2014 FR 1460810
(43) Date de publication de la demande: 11.05.2016
(73) Titulaire: Zenou, Bernard, 93340 Le Raincy (FR)
(72) Inventeur: ZENOU, Bernard, 93340 Le Raincy (FR); ZENOU, Laurent, 92300 Levallois-Perret (FR)
(74) Mandataire: Lenne, Laurence

(56) Documents cités:
- JP-A- 2013 052 021
- US-A- 4 869 733
- US-A- 5 137 549
- US-A- 5 226 933
- US-A- 5 529 607
- US-A- 5 917 135
- US-A1- 2006 162 565
- US-A1- 2011 209 707

## Description

L'invention concerne un dispositif de production d'oxygène purifié.

Elle se rapporte plus précisément à un dispositif de production d'oxygène purifié, par le procédé de production d'oxygène appelé « PSA » (acronyme de « Pressure Swing Adsorption ») qui consiste à séparer les molécules d'azote contenues dans l'air en faisant traverser l'air dans une colonne ou lit contenant un tamis moléculaire, le plus souvent de type zéolite, qui est un matériau ayant la capacité d'adsorber les molécules d'azote, en faisant varier la pression dans un cycle d'adsorption/désorption.

Bien que pouvant être conçu avec un seul lit, ce type de dispositif comporte souvent deux lits qui travaillent alternativement en adsorption/désorption permettant de produire à chaque cycle un certain volume d'oxygène avec une concentration comprise entre 90% et 96%, les autres constituants étant principalement de l'argon et des résidus d'azote.

Le plus souvent, les phases d'un cycle PSA sont pressurisation/production/égalisation/dépressurisation/régénération.

Un second étage peut être ajouté à un tel dispositif pour obtenir une pureté supérieure d'oxygène. Son rôle est de séparer le mélange d'oxygène, d'argon, de résidus d'azote et de quelques gaz rares pour en extraire un oxygène de haute pureté pouvant atteindre 99,5%.

Fonctionnant aussi selon le procédé « PSA », le second étage comporte un ou deux lits de tamis moléculaire, par exemple un tamis moléculaire au carbone, qui a la propriété d'adsorber l'oxygène. L'opération consiste à faire passer le mélange gazeux dans le lit et d'évacuer à l'atmosphère la partie du gaz non adsorbé, à savoir essentiellement de l'argon et des résidus d'azote.

Un tel dispositif est décrit dans le document de brevet US 5 137 549.

Le concentrateur d'oxygène à deux étages qui y est décrit comporte un premier étage de séparation séparant l'azote, le dioxyde de carbone et la vapeur d'eau de l'air. Le mélange d'oxygène et d'argon produit par ce premier étage est injecté par l'intermédiaire d'une vanne d'alimentation dans un second étage de séparation qui comporte deux lits de matériau d'adsorption de l'oxygène, une purge d'évacuation de l'argon séparé, cette purge étant équipé d'une vanne de purge, et un circuit d'injection d'une partie de l'oxygène purifié produit, dans l'alimentation en mélange d'oxygène et d'argon au niveau de la vanne d'alimentation.

La commande de la vanne de purge est effectuée de façon décalée et alternée de la commande de la vanne d'alimentation qui quant à elle est effectuée en même temps qu'une vanne de transfert qui est ouverte ou fermée pour connecter les deux lits en série.

Par ailleurs, ce type de dispositif de production d'oxygène purifié à double étage est destiné à produire un oxygène très purifié et ces dispositifs sont construits avec des paramètres fixes définis à la conception.

A titre d'exemple, si un premier étage peut produire un oxygène purifié à 93% ou 95% avec un débit de l'ordre de 10 m³/h, un second étage peut produire un oxygène purifié à 99% +/- 0,2% avec un débit de l'ordre de 5 m³/h.

Or il s'avère que le besoin des utilisateurs peut différer quant au taux de pureté de l'oxygène et/ou quant au débit de production d'oxygène purifié produit. Aucune solution n'est actuellement prévue pour répondre à ces besoins spécifiques différents ou pour optimiser les performances relatives de pureté de l'oxygène produit et de débit de production, pendant le fonctionnement.

L'invention résout ce problème en proposant un dispositif de production d'oxygène purifié, destiné en particulier à constituer le second étage d'un appareil de production d'oxygène appliquant le procédé PSA, qui soit particulièrement efficace et de meilleur rendement énergétique.

Pour ce faire, l'invention propose un dispositif de production d'oxygène purifié, pourvu d'une alimentation en mélange d'oxygène et d'argon, et comportant au moins un lit de matériau d'adsorption de l'oxygène, une purge d'évacuation de l'argon séparé et un circuit d'injection d'une partie de l'oxygène purifié produit, dans ladite alimentation, caractérisé en ce qu'il comporte un automate programmable de traitement du degré de pureté et/ou du débit de production paramétrables par l'utilisateur et un asservissement de ladite purge en fonction du degré de pureté de l'oxygène purifié et/ou du débit de production souhaités par l'utilisateur.

Le dispositif conforme à l'invention permet une interactivité avec l'utilisateur par le choix de la pureté d'oxygène qui peut s'effectuer sur l'interface homme/machine.

En mode automatique, le dispositif peut ajuster automatiquement son fonctionnement et par conséquent le taux de pureté d'oxygène, en fonction du débit d'oxygène requis par l'utilisateur.

De préférence, l'asservissement de la dite purge consiste à l'actionner en même temps que l'injection d'oxygène purifié, dans ladite alimentation, avec un volume de purge et d'injection variable en fonction de la commande reçue dudit automate.

Le dispositif peut comporter un analyseur, un débitmètre et/ou un capteur de pression de l'oxygène purifié assurant le contrôle dudit actionnement et connecté audit automate.

Il peut également comporter des tables de corrélation du taux de pureté, du débit de la purge et du volume de ladite purge assurant la commande dudit automate, ces tables de corrélation étant préalablement enregistrées dans l'automate.

De préférence, le dispositif comporte deux lits de matériau d'adsorption de l'oxygène fonctionnant en cycles selon les phases suivantes : pressurisation, purge/injection, égalisation et dépressurisation.

L'invention concerne également un appareil de production d'oxygène à partir d'air comportant un premier étage de séparation de l'azote et un second étage de séparation de l'argon constitué d'un dispositif tel que précisé ci-dessus.

L'invention est décrite ci-après plus en détail à l'aide de figures ne représentant que des modes de réalisation préférés de l'invention.
La figure 1 est une vue schématique d'un dispositif conforme à l'invention comportant un seul lit d'adsorption de l'oxygène.
La figure 2 est une vue schématique d'un dispositif conforme à l'invention comportant deux lits d'adsorption de l'oxygène.
La figure 3 est un diagramme de fonctionnement de ce dispositif.

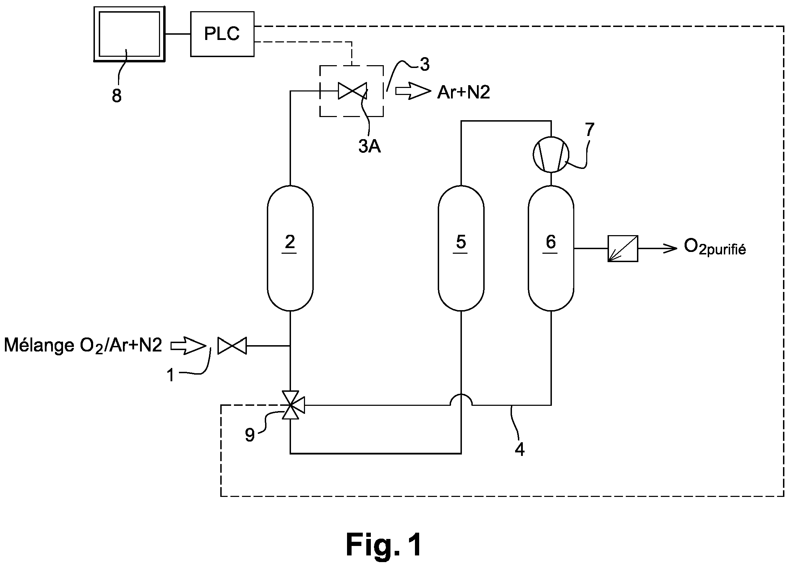

Comme illustré sur la figure 1, un dispositif de production d'oxygène purifié, est pourvu d'une alimentation 1 en mélange d'oxygène et d'argon, et comporte un lit 2 de matériau d'adsorption de l'oxygène, une purge 3 d'évacuation de l'argon séparé et un circuit 4 d'injection d'une partie de l'oxygène purifié produit, dans l'alimentation 1.

Le mélange d'oxygène et d'argon avec éventuellement quelques traces d'azote, provenant de préférence d'un premier étage de séparation de l'azote de l'air, est alimenté dans le lit 2 contenant un matériau d'adsorption de l'oxygène qui, lors de la traversée par le mélange, retient l'oxygène et libère l'argon et l'azote en partie haute. Cet argon est évacué à l'atmosphère par la purge 3 équipée d'une vanne de purge 3A.

En partie basse, par actionnement d'une vanne à trois voies 9, est évacué l'oxygène purifié qui est dépressurisé dans un réservoir 5 puis stocké dans un réservoir 6 par l'intermédiaire d'un surpresseur 7. De ce réservoir est produit l'oxygène purifié à un certain débit.

Par une conduite 4 de ce réservoir, une partie de l'oxygène purifié peut être réinjecté dans le mélange d'alimentation d'oxygène et d'argon et traité avec ce mélange dans le lit 2 pressurisé, par commande de la vanne trois voies 9.

Selon l'invention, ce dispositif comporte un automate programmable PLC de traitement du degré de pureté et/ou du débit de production paramétrables par l'utilisateur, qui peut entrer ces données sur un écran de contrôle 8.

La vanne de purge 3 est actionnée en même temps que cette vanne 9 d'injection d'oxygène purifié dans le mélange d'oxygène et d'argon traité, avec un volume de purge et d'injection variable en fonction de la commande reçue dudit automate.

Cet automate PLC comporte un asservissement de la vanne de purge 3A en fonction du degré de pureté de l'oxygène purifié et/ou du débit souhaités par l'utilisateur.

Cet asservissement peut être effectué au moyen d'un analyseur, d'un débitmètre et/ou d'un capteur de pression de l'oxygène purifié assurant le contrôle dudit actionnement et connecté à l'automate.

Il peut également être réalisé au moyen de tables de corrélation du taux de pureté, du débit de la purge et du volume de la purge assurant la commande de l'automate, ces tables de corrélation étant préalablement enregistrées dans l'automate.

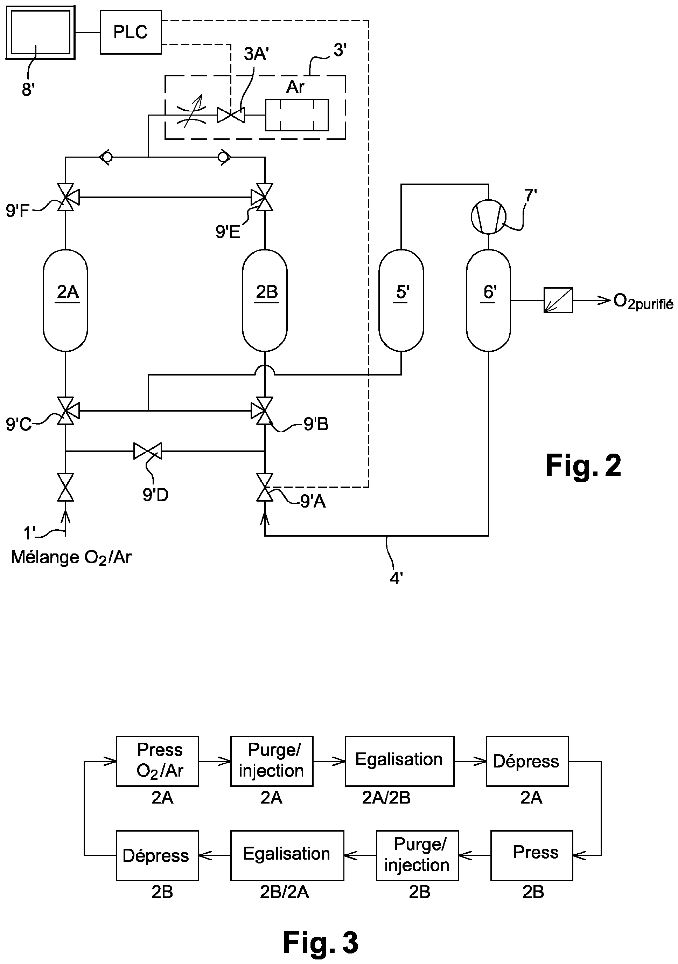

Sur le même principe, l'invention peut être appliquée à un dispositif de production d'oxygène comportant deux lits 2A, 2B de matériau d'adsorption de l'oxygène comme illustré sur les figures 2 et 3.

Le dispositif de production d'oxygène purifié est alors pourvu d'une alimentation 1' en mélange d'oxygène et d'argon, et comporte deux lits 2A, 2B de matériau d'adsorption de l'oxygène, une purge 3' d'évacuation de l'argon séparé et un circuit 4' d'injection d'une partie de l'oxygène purifié produit dans ladite alimentation 1'.

Le fonctionnement de ce dispositif est illustré sur la figure 3.

Le mélange d'oxygène et d'argon avec éventuellement quelques traces d'azote, provenant de préférence d'un premier étage de séparation de l'azote de l'air, est alimenté dans le premier lit 2A pressurisé contenant un matériau d'adsorption de l'oxygène qui, lors de la traversée par le mélange, retient l'oxygène et libère l'argon et des traces d'azote en partie haute. Cet argon est évacué à l'atmosphère par la purge 3' équipée d'une vanne de purge 3'A.

Par un agencement de vannes trois voies 9'B, 9'C, 9'E, 9'F, l'oxygène produit est alors transmis dans le second lit 2B pour égalisation des pressions des deux lits, dépressuration du premier lit 2A et production d'oxygène purifié, puis le second lit 2B est pressurisé par alimentation du mélange d'oxygène et d'argon et ce second lit 2B contient également un matériau d'adsorption de l'oxygène qui, lors de la traversée par le mélange, retient l'oxygène et libère l'argon et des traces d'azote en partie haute. Cet argon est évacué à l'atmosphère par la purge 3'.

L'oxygène produit est alors transmis dans le premier lit 2A pour égalisation des pressions des deux lits, dépressuration du second lit 2B et production d'oxygène purifié, et le traitement est poursuivi en cycles alternés.

L'oxygène purifié produit est dépressurisé dans un réservoir 5' puis stocké dans un réservoir 6' par l'intermédiaire d'un surpresseur 7'. De ce réservoir est produit l'oxygène purifié à un certain débit.

Par une conduite 4' de ce réservoir, une partie de l'oxygène purifié peut être réinjecté par l'intermédiaire de vannes 9'A, 9'D dans le mélange d'alimentation d'oxygène et d'argon 1' et traité avec ce mélange dans les lits pressurisés.

Selon l'invention, ce dispositif comporte un automate programmable PLC, qui outre la commande des diverses vannes du dispositif pour la production des cycles de production d'oxygène purifié, assure le contrôle du degré de pureté et/ou du débit paramétrables par l'utilisateur, qui peut entrer ces données sur un écran de contrôle 8'.

La vanne de purge 3' est actionnée en même temps que l'oxygène purifié est injecté au moyen de la vanne 9'A dans ladite alimentation 1', avec un volume de la purge et une injection de l'oxygène purifié variable, en fonction de la commande reçue de l'automate.

Cet automate PLC comporte un asservissement de la vanne de purge 3'A en fonction du degré de pureté de l'oxygène purifié et/ou du débit souhaités par l'utilisateur, en fonction du mode de régulation choisi par l'utilisateur.

Comme précédemment, cet asservissement peut être effectué au moyen d'un analyseur, d'un débitmètre et/ou d'un capteur de pression de l'oxygène purifié assurant le contrôle de l'actionnement et connecté à l'automate.

Il peut également être réalisé au moyen de tables de corrélation du taux de pureté, du débit de la purge et du volume de la purge assurant la commande de l'automate, ces tables de corrélation étant préalablement enregistrées dans l'automate.

## Revendications

1. Dispositif de production d'oxygène purifié, pourvu d'une alimentation (1, 1') en mélange d'oxygène et d'argon, et comportant au moins un lit (2, 2A, 2B) de matériau d'adsorption de l'oxygène, une purge (3, 3') d'évacuation de l'argon séparé et un circuit (4, 4') d'injection d'une partie de l'oxygène purifié produit, dans ladite alimentation (1, 1'), **caractérisé en ce qu'**il comporte un automate (PLC) programmable de traitement du degré de pureté et/ou du débit de production paramétrables par l'utilisateur, comportant un asservissement de ladite purge (3, 3') en fonction du degré de pureté de l'oxygène purifié et/ou du débit de production souhaités par l'utilisateur.

2. Dispositif selon la revendication précédente, **caractérisé en ce que** l'asservissement de la dite purge (3, 3') consiste à l'actionner en même temps que l'injection d'oxygène purifié dans ladite alimentation (1, 1'), avec un volume de ladite purge et une injection de l'oxygène purifié variable, en fonction de la commande reçue du dit automate.

3. Dispositif selon la revendication précédente, **caractérisé en ce qu'**il comporte un analyseur de l'oxygène purifié assurant le contrôle dudit actionnement et connecté audit automate.

4. Dispositif selon la revendication 2 ou 3, **caractérisé en ce qu'**il comporte un débitmètre de l'oxygène purifié assurant le contrôle dudit actionnement et connecté audit automate.

5. Dispositif selon l'une des revendications précédentes 2 à 4, **caractérisé en ce qu'**il comporte un capteur de pression de l'oxygène purifié assurant le contrôle dudit asservissement et connecté audit automate.

6. Dispositif selon l'une des revendications 2 à 5, **caractérisé en ce qu'**il comporte des tables de corrélation du taux de pureté, du débit de la purge (3, 3') et du volume de ladite purge assurant la commande dudit automate (PLC), ces tables de corrélation étant préalablement enregistrées dans l'automate (PLC).

7. Dispositif selon l'une des revendications précédentes, **caractérisé en ce qu'**il comporte deux lits (2A, 2B) de matériau d'adsorption de l'oxygène fonctionnant en cycles selon les phases suivantes : pressurisation, purge/injection, égalisation et dépressurisation.

8. Appareil de production d'oxygène à partir d'air comportant un premier étage de séparation de l'azote et un second étage de séparation de l'argon constitué d'un dispositif selon l'une des revendications précédentes.

## Patentansprüche

1. Vorrichtung für die Herstellung von gereinigtem Sauerstoff, versehen mit einer Versorgung (1, 1') mit Sauerstoff-Argon-Gemisch und aufweisend mindestens ein Bett (2, 2A, 2B) aus Adsorptionsmaterial von Sauerstoff, eine Entlüftung (3, 3') zum Ablassen des separierten Argons und einen Kreis (4, 4') zum Einleiten eines Teils des hergestellten gereinigten Sauerstoffs in die Versorgung (1, 1'), **dadurch gekennzeichnet, dass** sie eine programmierbare Automatik (PLC) für die Bearbeitung des von dem Benutzer einstellbaren Reinheitsgrads und/oder des Produktionsdurchsatzes aufweist, aufweisend eine Regelung der Entlüftung (3, 3') in Abhängigkeit des von dem Benutzer gewünschten Reinheitsgrads des gereinigten Sauerstoffs und/oder des Produktionsdurchsatzes.

2. Vorrichtung nach vorangehendem Anspruch, **dadurch gekennzeichnet, dass** die Regelung der Entlüftung (3, 3') darin besteht, sie gleichzeitigt wie das Einleiten von gereinigtem Sauerstoff in die Versorgung (1, 1') mit einem variablen Volumen der Entlüftung und einer Einleitung von gereinigtem Sauerstoff in Abhängigkeit von dem von der Steuerung erhaltenen Befehl zu betätigen.

3. Vorrichtung nach vorangehendem Anspruch, **dadurch gekennzeichnet, dass** sie einen Analysator des gereinigten Sauerstoffs aufweist, welcher die Kontrolle der Betätigung gewährleistet und mit der Steuerung verbunden ist.

4. Vorrichtung nach Anspruch 2 oder 3, **dadurch gekennzeichnet, dass** sie einen Durchflussmesser des gereinigten Sauerstoffs aufweist, welcher die Kontrolle der Betätigung gewährleistet und mit der Automatik verbunden ist.

5. Vorrichtung nach einem der Ansprüche 2 bis 4, **dadurch gekennzeichnet, dass** sie einen Drucksensor des gereinigten Sauerstoffs aufweist, welcher die Kontrolle der Betätigung gewährleistet und mit der Automatik verbunden ist.

6. Vorrichtung nach einem der Ansprüche 2 bis 5, **dadurch gekennzeichnet, dass** sie Korrelationstabellen des Reinheitsgrads, des Entlüftungsdurchsatzes (3, 3') und des Volumens der Entlüftung zwecks Sicherung der Steuerung der Automatik (PLC) aufweist, wobei diese Korrelationstabellen zuvor in der Automatik (PLC) gespeichert wurden.

7. Vorrichtung nach einem der vorangehenden Ansprüche, **dadurch gekennzeichnet, dass** sie zwei Betten (2A, 2B) aus Adsorptionsmaterial von Sauerstoff aufweist, die in Zyklen gemäß den folgenden Phasen arbeiten: Druckbeaufschlagung, Entlüftung/Einleitung, Ausgleich und Druckentlastung.

8. Apparat für die Herstellung von Sauerstoff aus Luft, aufweisend eine erste Separationsstufe des Stickstoffs und eine zweite Separationsstufe des Argons, gebildet von einer Vorrichtung nach einem der vorangehenden Ansprüche.

## Claims

1. Device for producing purified oxygen, provided with a feed (1, 1') of a mixture of oxygen and argon, and comprising at least one bed (2, 2A, 2B) of oxygen adsorbing material, a purge (3, 3') to evacuate the separated argon and a circuit (4, 4') to inject part of the produced purified oxygen into said feed (1, 1'), **characterized in that** it comprises a programmable logic controller (PLC) for user-parameterizable treatment of degree of purity and/or production flow rate, comprising a servo system of said purge (3, 3') as a function of the degree of purity of the purified oxygen and/or the production flow rate desired by the user.

2. The device according to the preceding claim, **characterized in that** the servo system of said purge (3, 3') consists of actuation thereof at the same time as the injection of purified oxygen into said feed (1, 1'), with a variable volume of said purge and injection of purified oxygen as a function of the instruction received from said controller.

3. The device according to the preceding claim, **characterized in that** it comprises an analyser of purified oxygen ensuring the control of said actuation and connected to said controller.

4. The device according to claim 2 or 3, **characterized in that** it comprises a flowmeter of purified oxygen ensuring the control of said actuation and connected to said controller.

5. The device according to one of the preceding claims 2 to 4, **characterized in that** it comprises a pressure sensor of the purified oxygen ensuring the control of said servo system and connected to said controller.

6. The device according to one of claims 2 to 5, **characterized in that** it comprises correlation tables of degree of purity, flow rate of purge (3, 3') and volume of said purge ensuring the controlling by said controller (PLC), these correlation tables being previously recorded in the controller (PLC).

7. The device according to one of the preceding claims, **characterized in that** it comprises two beds (2A, 2B) of oxygen adsorbing material operating in cycles with the following phases: pressurization, purge/injection equalization and depressurization.

8. Equipment for producing oxygen from air comprising a first nitrogen separation stage and a second argon separation stage, formed of a device according to one of the preceding claims.
